(19) Europäisches Patentamt
European Patent Office
Office européen des brevets

(11) Publication number: **0 430 036 A2**

(12) # EUROPEAN PATENT APPLICATION

(21) Application number: **90122182.0**

(22) Date of filing: **20.11.90**

(51) Int. Cl.[5]: **A61K 31/55**, //(A61K31/55, 31:475),(A61K31/55,31:71)

(30) Priority: **22.11.89 US 440121**

(43) Date of publication of application: **05.06.91 Bulletin 91/23**

(84) Designated Contracting States: **AT BE CH DE DK ES FR GB GR IT LI LU NL SE Bulletin**

(71) Applicant: **MARION MERRELL DOW INC.**
**9300 Ward Parkway**
**Kansas City, Missouri 64114(US)**

(72) Inventor: **Ahmed, Nahed K.**
**8812 Rosewood**
**Prairie Village, Kansas 66207(US)**

(74) Representative: **Vossius & Partner**
**Siebertstrasse 4 P.O. Box 86 07 67**
**W-8000 München 86(DE)**

(54) **Benzothiazepine metastasis treatment.**

(57) Metastasis is treated with certain benzothiazepines.

EP 0 430 036 A2

## BENZOTHIAZEPINE METASTASIS TREATMENT

This invention is directed to the use of benzothiazepines for cancer metastasis treatment.

Metastasis, in cancer, the appearance of neoplasms in parts of the body remote from the seat of the primary tumor, is a problem recurring in cancer treatment. It results from dissemination of tumor cells by the lymphatics or blood vessels, or through serous cavities or subarachnoid or other spaces, and if widespread, usually renders the cancer incurable by surgery alone.

Some literature in the art reports that calcium channel blockers may represent a new class of antimetastatic agents with several attractive features, including low chronic toxicity, oral administration capability, clinical trial stage research, and/or already approved for cardiovascular disease. See e.g., Honn et al., Biochem. Pharm. **34**:235-41 (1985); Tsuruo et al., Cancer Chemother. Pharmacol. **14**:30-3 (1985); Onoda et al., Cancer Lett. **30**:181-8 (1986); Onoda et al., Proc. Annu. Meet. Am. Assoc. Cancer Res. **25**:351 (1984); Honn et al., Proc. Soc. Exp. Biol. Med. **174**:16-9 (1983); Konn et al., "Treatment of Metastasis: Problems and Prospects," London, Taylor and Francis, (1984) pp. 259-62;

Pauwels-Vergely et al., Fourth European Conference on Clinical Oncology and Cancer Nursing, Madrid, Nov. 1-4, 1987, Federation of European Cancer Societies, 1987, p. 88.

European Patent Application EP (our ref.: A 637 EP) entitled "ANTI-CANCER DRUG POTENTIATORS," filed on even date herewith, discloses that certain benzothiazepines are cancer drug potentiators.

The art lacks and needs new treatments for metastasis.

This invention provides for the use of a benzothiazepine for preparing a pharmaceutical composition for controlling cancer metastasis in an organism having a cancer.

The present invention is useful in cancer treatment. More particularly, this invention may control cancer metastasis to a notably effective degree. The benzothiazepine is hereinafter referred to as "potentiator drug".

In the practice of this invention, the Benzothiazepine-potentiator drug is administered to the organism in which the cancer metastasis is desired to be controlled. The organism may be a mammal to include a human patient.

The term "control" refers herein to a regulation. The regulation may be by partial or total cessation.

The term "administering" and so forth refers herein to suitably providing an amount of compound or composition the organism so that the desired therapeutic effect might result. The administering can be by conventional dosage formats. The conventional dosage formats include the ingestion of a powder, beads, tablet, capsule, syrup or dragee, the injection of an intravenous solution or colloidal mixture, the implantation of a sustainably releasing article or device, the application of an ointment such as a creme or a gel, and the transdermal application of components with the aid of a suitable carrier.

The term "Benzothiazepine-potentiator drug" refers herein to a benzothiazepine compound to include suitable pharmaceutically acceptable salts thereof, which excludes diltiazem, i.e., d-cis-5-[2-(dimethylamino)ethyl]-2,3-dihydro-3-hydroxy-2-(4-methoxyphenyl)-1,5-benzothiazepin-4(5H)-one acetate (ester), and its pharmaceutically acceptable salts, e.g., its hydrochloride, and which potentiates the anti-cancer drug effect of an anti-cancer drug administered in general concurrence therewith. Desirably, other therapeutic effect(s) such as at least one of anti-hypertensive effect, anti-convulsant effect, and anti-depressant effect are substantially low or absent in the practice of this invention with respect to the Benzothiazepine-potentiator drug in particular.

The Benzothiazepine-potentiator drug can be selected from a compound represented by the following general formula:

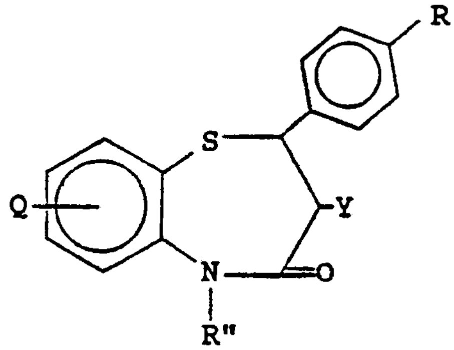

(I)

wherein

Q is hydro (H) or halo to include fluoro (F) and chloro (Cl), especially H or 8-Cl;

R is H, lower alkoxy, lower haloalkyl, cyano (CN), lower alkyl to include methyl (CH3) or halo to include F & Cl, especially H, methoxy (OMe), trifluoromethyl (CF3) or CN;

Y is

OR′, wherein R′ is H or alkylacyl to include, e.g., lower alkylacyl and adamantylcarboxy, etc., especially H, or lower alkylacyl to include groups such as acetyl, propionyl, butyryl, pivalyl, valeryl, isovaleryl, etc., provided that then there is full saturation between carbons 2 & 3 of the benzothiazepine nucleus and 2,3-dihydro-functional ity thereat as well, or

Cl, provided that then there is ethylenic unsaturation between positions 2 & 3 of the benzothiazepine nucleus, and

R″ is 2-[di(lower alkyl)amino]ethyl (R″1),

3-[di(lower alkyl)amino]propyl (R″2), 2-(pyrrolidino)ethyl (R″3), 3-(pyrrolidino)propyl (R″4), 2-(piperidino)ethyl (R″5), 3-(piperidino)propyl (R″6), 2-(morpholino)ethyl (R″7), 3- (morhpolino)propyl (R″8) or (N-pyridinium)-alkyl with a suitable counterion being present (+R″9-X), especially R″1, e.g., with R″1 being 2-(dimethylamino)ethyl (R″1a) or with R″1 being 2-(diisopropylamino)ethyl (R″1b), or R″3, or R″5, or +R″9-X, e.g., with +R″9-X being 2-(N-pyridinium) ethyl with a bromide and/or chloride counterion being present (+R″9a-X).

Generally, with respect to the 2,3-dihydro compounds, the cis-configuration, i.e., about positions 2 & 3 of the benzothiazepine nucleus, is or can be present, with some exceptions to this, e.g., with the trans-configuration, i.e., about positions 2 & 3 of the benzothiazepine nucleus, being present in the cases of Q being H or 8-Cl, R being methoxy, R′ being H, and R″ being R″5. Also generally, if the compound has a chiral carbon, racemates or separate optical antipodes may be present, with some exceptions to this, e.g., the levorotary optical antipode corresponding to diltiazem being one such exception; a compound employable in the practice of this invention.

Suitable pharmaceutically acceptable salts generally include the hydrochloride, the fumerate, the maleate, the sulfate, the citrate, and so forth.

The Benzothiazepine-potentiator drugs can be made by reacting a suitable glycidic acid ester with a suitable aminothiophenol to prepare corresponding aminophenylthiopropionic acid ester, then cyclyzing the latter ester or its corresponding free acid, followed by N-alkylation and 3-acylation as may be desired; see e.g. US-A-3,562,257, 4,567,175 and 4,885,375 as well as European Patent Application EP (our ref.: A 635 EP) entitled "BENZOTHIAZEPINES", and filed on even date herewith. In addition, the Benzothiazepine-potentiator drugs having the vinyl chloride at positions 2 & 3 of the benzothiazepine nucleus can be made by processes known in the art; see e.g. US-A-3,895,006, 3,983,106 and 3,075,967.

The Benzothiazepine-potentiator drug may be used alone. Alternatively, it may be administered in combination with, to include in general concurrence with, other drugs, especially for example, an anti-cancer drug.

The anti-cancer drug may desirably be an anthracycline, e.g., daunorubicin, and/or a vinca alkaloid, e.g., vincristine or vinblastin. It may also be a cis-platinum compound.

In general, the better potentiating effect a combination has, the more likely it is that this combination will be correspondingly better in controlling cancer metastasis. Therefore, further and particular reference to the Example with Comparative of the European Patent Application EP (our ref.: A 637 EP), is hereby made for the selection of superiorly performing combinations that with reasonable assurance would provide superior control of cancer metastasis in accordance with the practice of this invention. Thus, in general, the Benzothiazepine- potentiator (B-P) drug may be at least one B-P drug, or a pharmaceutically acceptable

salt thereof, selected from the the following B-P drugs, identified in Table I as follows with respect to compounds of the formula I:

TABLE I

| B-P drug | O | R | Y: $R'$ of $OR'$ &c | $R''$ |
|---|---|---|---|---|
| 1-cis-DTZ | H | OMe | acetyl | $R''1a$ |
| cis-ML1013 | H | OMe | valeryl | $R''1a$ |
| cis-ML1014 | H | OMe | isovaleryl | $R''1a$ |
| cis-ML1015 | H | OMe | pivalyl | $R''1a$ |
| cis-ML1016 | H | OMe | acetyl | $R''1b$ |
| cis-ML1017 | H | OMe | H | $R''5$ |
| cis-ML1018 | H | OMe | acetyl | $R''5$ |
| cis-ML1020 | H | OMe | H | $R''3$ |
| cis-ML1021 | H | OMe | acetyl | $R''3$ |
| cis-ML1047 | 8-Cl | OMe | pivalyl | $R''1a$ |
| cis-ML1048 | H | OMe | H | $R''6$ |
| cis-ML1063 | H | OMe | H | $R''7$ |
| cis-ML1064 | H | OMe | acetyl | $R''7$ |
| cis-ML1065 | H | OMe | H | $+R''9a$-X |
| cis-ML1066 | H | OMe | acetyl | $+R''9a$-X |
| cis-ML1077 | H | Cl | H | $R''1a$ |
| cis-ML1078 | H | CF3 | H | $R''1a$ |
| cis-ML1079 | H | CH3 | H | $R''1a$ |
| cis-ML1080 | H | OMe | adamantylcarboxy | $R''1a$ |
| cis-ML1082 | H | CF3 | H | $R''5$ |
| trans-ML1096 | 8-Cl | OMe | H | $R''5$ |
| ML1097 | H | OMe | *Cl | $R''1a$ |
| cis-ML1098 | H | H | H | $R''5$ |
| trans-ML1103 | H | OMe | H | $R''5$ |
| cis-ML1104 | H | CN | H | $R''5$ |
| cis-TA3090 | 8-Cl | OMe | acetyl | $R''1a$ |

(*vinyl chloride at positions 2,3)

A suitable dosage or dosage regimen is employed according to the desired effect of the component(s) employed in relation to the cancer and the organism.

By the practice of this invention, cancer metastasis can be generally controlled. Preferably, the control is of a substantial nature.

## Claims

1. The use of a benzothiazepine for preparing a pharmaceutical composition for controlling cancer metastasis in an organism having a cencer.
2. The use of claim 1, wherein said benzothiazepine is selected from a compound represented by the following general formula:

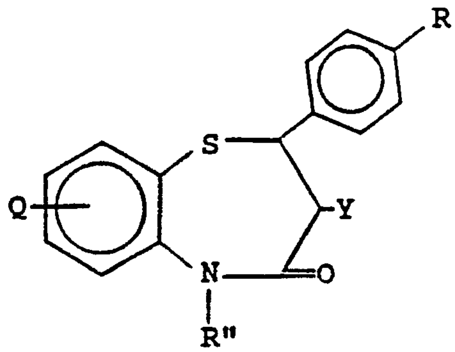

(I)

wherein
Q is H or halo;
R is H, lower alkoxy, lower haloalkyl, CN, lower alkyl or halo;
Y is
OR′, wherein R′ is H or alkylacyl, provided that then there is full saturation between carbons 2 & 3 of the benzothiazepine nucleus and 2,3-dihydro-functionality thereat as well, or
Cl, provided that then there is ethylenic unsaturation between positions 2 & 3 of the benzothiazepine nucleus, and
R″ is 2-[di(lower alkyl)amino]ethyl (R″1), 3-[di(lower alkyl)amino]propyl (R″2), 2- (pyrrolidino)ethyl (R″3), 3-(pyrrolidino)propyl (R″4), 2-(piperidino)ethyl (R″5), 3-(piperidino)propyl (R″6), 2-(morpholino)ethyl (R″7), 3-(morhpolino)propyl (R″8) or (N-pyridinium)alkyl with a suitable counterion being present (+R″9-X).

3. The use of claim 2, wherein said benzothiazepine is at least one B-P drug, or a pharmaceutically acceptable salt thereof, selected from the group consisting of the following B-P drugs, identified as follows with respect to compounds of the formula I:

| B-P drug | O | R | Y: R′ of OR′ &c | R″ |
|---|---|---|---|---|
| 1-cis-DTZ | H | OMe | acetyl | R″1a, |
| cis-ML1013 | H | OMe | valeryl | R″1a, |
| cis-ML1014 | H | OMe | isovaleryl | R″1a, |
| cis-ML1015 | H | OMe | pivalyl | R″1a, |
| cis-ML1016 | H | OMe | acetyl | R″1b, |
| cis-ML1017 | H | OMe | H | R″5, |
| cis-ML1018 | H | OMe | acetyl | R″5, |
| cis-ML1020 | H | OMe | H | R″3, |
| cis-ML1021 | H | OMe | acetyl | R″3, |
| cis-ML1047 | 8-Cl | OMe | pivalyl | R″1a, |
| ois-ML1048 | H | OMe | H | R″6, |
| cis-ML1063 | H | OMe | H | R″7, |
| cis-ML1064 | H | OMe | acetyl | R″7, |
| cis-ML1065 | H | OMe | H | +R″9a-X, |
| cis-ML1066 | H | OMe | acetyl | +R″9a-X, |
| cis-ML1077 | H | Cl | H | R″1a, |
| cis-ML1078 | H | CF3 | H | R″1a, |
| cis-ML1079 | H | CH3 | H | R″1a, |
| cis-ML1080 | H | OMe | adamantylcarboxy | R″1a, |
| cis-ML1082 | H | CF3 | H | R″5, |
| trans-ML1096 | 8-Cl | OMe | H | R″5, |
| ML1097 | H | OMe | *Cl | R″1a, |
| cis-ML1098 | H | H | H | R″5, |
| trans-ML1103 | H | OMe | H | R″5, |
| cis-ML1104 | H | CN | H | R″5 and |
| cis-TA3090 | 8-Cl | OMe | acetyl | R″1a, |

(*vinyl chloride at positions 2,3)

further wherein
OMe is methoxy;
CF3 is trifluoromethyl;
CH3 is methyl;
R″1a is 2-(dimethylamino)ethyl;
R″1b is (diisopropylamino)ethyl, and
+R″9a-X is 2-(N-pyridinium)ethyl with a bromide and/or chloride counterion being present.

4. The use of claim 1, wherein an anti-cancer drug is prepared in combination with the benzothiazepine.

5. The use of claim 4, wherein the anti-cancer drug is at least one of an anthracycline or a vinca alkaloid.

6. The use of claim 5, wherein the anthracycline is daunorubicin, and the vinca alkaloid is vincristine or vinblastin.

7. The use of any one of claims 1 to 6, wherein said benzothiazepine is 1-cis-5-[2-(dimethylamino)ethyl]-2,3-dihydro-3-hydroxy-2-(4-methoxyphenyl)-1,5-benzothiazepin-4(5H)-one acetate (ester) or a pharmaceutically acceptable salt thereof.

8. The use of any one of claims 1 to 6, wherein said benzothiazepine is selected from at least one of the group consisting of:
1-cis-8-chloro-5-[2-(dimethylamino)ethyl]-2,3-dihydro-3-hydroxy-2-(4-methoxyphenyl)-1,5-benzothiazepin-4-(5H)-one acetate (ester);
d-cis-5-[2-(dimethylamino)ethyl]-2,3-dihydro-3-hydroxy-2-(4-methoxyphenyl)-1,5-benzothiazepin-4(5H)-one valerylate (ester);
d-cis-5-[2-(dimethylamino)ethyl]-2,3-dihydro-3-hydroxy-2-(4-methoxyphenyl)-1,5-benzothiazepin-4(5H)-one isovalerylate (ester);
d-cis-5-[2-(dimethylamino)ethyl]-2,3-dihydro-3-hydroxy-2-(4-methoxyphenyl)- 1,5-benzothiazepin-4(5H)-one pivalate (ester);
d-cis-5-[2-(diisopropylamino)ethyl]-2,3-dihydro-3-hydroxy-2-(4-methoxyphenyl)-1,5-benzothiazepin-4(5H)-one acetate (ester);

dl-cis-5-{2-[(pyrrolidino)ethyl]amino}-2,3-dihydro-3-hydroxy-2-(4-methoxyphenyl)-1,5-benzothiazepin-4(5H)-one acetate (ester);
dl-cis-5-[2-(dimethylamino)ethyl]-2,3-dihydro-3-hydroxy-2-(4-trifluoromethylphenyl)-1,5-benzothiazepin-4(5H)-one;
d-cis-5-[2-(piperidino)ethyl]-2,3-dihydro-3-hydroxy-2-(4-trifluorophenyl)-1,5-benzothiazepin-4(5H)-one;
d-trans-5-[2-(piperidino)ethyl]-2,3-dihydro-3-hydroxy-2-(4-methoxyphenyl)-1,5-benzothiazepin-4(5H)-one;
d-cis-5-[2-(piperidino)ethyl]-2,3-dihydro-3-hydroxy-2-(4-cyanophenyl)-1,5-benzothiazepin-4(5H)-one, and pharmaceutically acceptable salt(s) thereof.

9. The use of claim 8, wherein said benzothiazepine is used together with daunorubicin.

10. The use of any one of claims 1 to 8, wherein said salt(s) of said benzothiazepines are the maleate salt or the hydrochloride salt.